(19) Europäisches Patentamt
European Patent Office
Office européen des brevets

(11) Veröffentlichungsnummer: **0 475 158 A2**

(12) **EUROPÄISCHE PATENTANMELDUNG**

(21) Anmeldenummer: **91114152.1**

(22) Anmeldetag: **23.08.91**

(51) Int. Cl.5: **A01N 37/36**, A01N 37/38, A01N 37/44, A01N 37/48, A01N 49/00

(30) Priorität: **14.09.90 DE 4029192**

(43) Veröffentlichungstag der Anmeldung: **18.03.92 Patentblatt 92/12**

(84) Benannte Vertragsstaaten: **AT BE CH DE DK ES FR GB IT LI NL**

(71) Anmelder: **BASF Aktiengesellschaft**
**Carl-Bosch-Strasse 38**
**W-6700 Ludwigshafen(DE)**

(72) Erfinder: **Kirstgen, Reinhard, Dr.**
**Erkenbrechtstrasse 23e**
**W-6730 Neustadt(DE)**
Erfinder: **Otter, Rainer, Dr.**
**Lessingstrasse 11**
**W-6947 Laudenbach(DE)**
Erfinder: **Kuenast, Christoph, Dr.**
**Salierstrasse 2**
**W-6701 Otterstadt(DE)**
Erfinder: **Kardorff, Uwe, Dr.**
**D 3,4**
**W-6800 Mannheim 1(DE)**
Erfinder: **Steglich, Wolfgang, Prof.Dr.**
**Hobsweg 77**
**W-5300 Bonn-Roettgen(DE)**
Erfinder: **Bertram, Gunda**
**Nesselroder Strasse 27**
**W-5300 Bonn 3(DE)**

(54) **Verwendung von alpha-Arylacrylsäurederivaten zur Bekämpfung von Schädlingen.**

(57) Verwendung von alpha-Arylacrylsäurederivaten der allgemeinen Formel I,

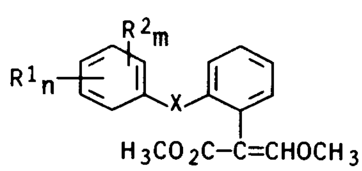

I

in der die Substituenten und Indizes folgende Bedeutung haben:

X

Ethylen oder Ethenylen;

n

0 oder 1;

m

0, 1, 2, 3 oder 4;

$R^1$

Cyano; Cyanato; Thiocyanato; Nitro; Hydroxy; Carboxyl; Halogenalkyl; Halogenalkoxy; ggf. subst. Cycloalkyl, Cycloalkenyl, Cycloalkdienyl, Alkenyl, Alkinyl oder Amino; Alkylcarbonyl; Alkoxycarbonyl; Alkylaminocarbonyl; Dialkylaminocarbonyl; ggf. subst. Benzyloxycarbonyl, Benzoyl, Phenylalkyl, Phenylalkoxy oder Phenoxyalkyl; oder $R^1$ gemeinsam mit einem der Reste $R^2$ eine ggf. subst. 1,3-Butadien-1,4-diylgruppe oder eine ggf. subst. Kette aus Kohlenstoffgliedern und einem Sauerstoffglied;

EP 0 475 158 A2

$R^2$
Halogen;
ggf. subst. Alkyl, Alkoxy, Alkenyloxy, Alkinyloxy, Alkdienyloxy, Benzyloxy oder Benzylthio
zur Bekämpfung von Schädlingen und Verfahren zu ihrer Verwendung.

Die vorliegende Erfindung betrifft die Verwendung von α-Arylacrylsäurederivaten der allgemeinen Formel I,

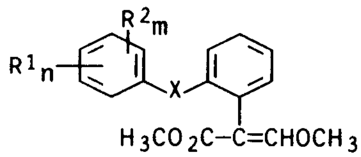

I

in der die Substituenten und Indizes folgende Bedeutung haben:

X

Ethylen oder Ethenylen;

n

0 oder 1;

m

0, 1, 2, 3 oder 4, wobei die Reste $R^2$ verschieden sein können, wenn m für 2, 3 oder 4 steht;

$R^1$

Cyano; Cyanato; Thiocyanato; Nitro; Hydroxy; Carboxyl;

$C_1$-$C_4$-Halogenalkyl; $C_1$-$C_4$-Halogenalkoxy;

$C_3$-$C_8$-Cycloalkyl, $C_5$-$C_8$-Cycloalkenyl oder $C_5$-$C_8$-Cycloalkdienyl, wobei diese Reste ein bis fünf Halogenatome, einen Phenyl-, Phenoxy- oder Phenylthiorest und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

$C_2$-$C_6$-Alkenyl oder $C_2$-$C_6$-Alkinyl, wobei diese Reste ein bis fünf Halogenatome, einen Phenyl-, Phenoxy- oder Phenylthiorest und/oder ein bis drei der folgenden Reste tragen können: $C_1$-$C_4$-Alkoxy, $C_1$-$C_4$-Halogenalkoxy, $C_1$-$C_4$-Alkylthio oder $C_1$-$C_4$-Halogenalkylthio;

Amino, welches eine $C_1$-$C_6$-Alkylcarbonyl-, $C_1$-$C_6$-Alkoxycarbonyl-, $C_1$-$C_6$-Alkylaminocarbonyl- oder Di-$C_1$-$C_6$-alkylaminocarbonylgruppe und/oder ein oder zwei $C_1$-$C_6$-Alkylgruppen tragen kann;

$C_1$-$C_6$-Alkylcarbonyl; $C_1$-$C_6$-Alkoxycarbonyl; $C_1$-$C_6$-Alkylaminocarbonyl; Di-$C_1$-$C_6$-alkylaminocarbonyl;

Benzyloxycarbonyl, Benzoyl, Phenyl-$C_1$-$C_4$-alkyl, Phenyl-$C_1$-$C_4$-alkoxy oder Phenoxy-$C_1$-$C_4$-alkyl, wobei die aromatischen Reste ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

oder $R^1$ gemeinsam mit einem der Reste $R^2$ in ortho-Position

eine 1,3-Butadien-1,4-diylgruppe, wobei dieser Rest ein bis vier Halogenatome und/oder ein oder zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

oder eine drei- oder viergliedrige Kette aus Kohlenstoffgliedern und einem Sauerstoffglied, wobei diese Kette ein bis drei $C_1$-$C_4$-Alkylgruppen tragen kann;

$R^2$

Halogen;

$C_1$-$C_{10}$-Alkyl, $C_1$-$C_6$-Alkoxy, $C_2$-$C_6$-Alkenyloxy, $C_2$-$C_6$-Alkinyloxy oder $C_5$-$C_{10}$-Alkdienyloxy, wobei diese Reste ein bis fünf Halogenatome, einen Phenyl-, Phenoxy- oder Phenylthiorest und/oder ein bis drei der folgenden Reste tragen können: $C_1$-$C_4$-Alkoxy, $C_1$-$C_4$-Halogenalkoxy, $C_1$-$C_4$-Alkylthio oder $C_1$-$C_4$-Halogenalkylthio;

Benzyloxy oder Benzylthio, wobei die aromatischen Reste ein bis fünf Halogenatome, einen Phenyl-, Phenoxy- oder Phenylthiorest und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

zur Bekämpfung von Schädlingen.

Außerdem betrifft die Erfindung Verfahren zur Bekämpfung von Schädlingen.

Aus der Literatur sind alpha-Arylacrylsäurederivate als Fungizide bekannt, deren allgemeine Formel die eingangs definierten Verbindungen der Formel I umfaßt (EP-A 203 606, EP-A 229 974). Außerdem sind α-Arylacrylsäurederivate als Insektizide und Fungizide (EP-A 178 826) und als Insektizide (EP-A 256 667, EP-A 335 519) bekannt.

Aufgabe der vorliegenden Erfindung waren neue wirksame Möglichkeiten zur Schädlingsbekämpfung.

Demgemäß wurde gefunden, daß die eingangs definierten α-Arylacrylsäurederivate I sich zur Bekämpfung von Schädlingen eignen.

Die Herstellung der α-Arylacrylsäurederivate I ist in der eingangs zitierten Literatur beschrieben (EP-A 203 606, EP-A 229 974).

Im Hinblick auf die bestimmungsgemäße Verwendung der Verbindungen I zur Bekämpfung von Schädlingen kommen als Substituenten und Indizes die Folgenden in Betracht:

X

Ethylen ($-CH_2-CH_2-$) oder Ethenylen ($-CH=CH-$);

n

0 oder 1;

m

0, 1, 2, 3 oder 4, wobei die Reste $R^2$ verschieden sein können, wenn m für 2, 3 oder 4 steht;

$R^1$

Cyano; Cyanato; Thiocyanato; Nitro; Hydroxy; Carboxyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trichlormethyl, Difluormethyl und 2,2-Difluorethyl, insbesondere Trifluormethyl und 2,2,2-Trifluorethyl;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Dichlormethyloxy, Trifluormethyloxy, Dichlorfluormethyloxy und Pentafluorethyloxy, insbesondere Difluormethyloxy, 2,2-Difluorethyloxy und 2,2,2-Trifluorethyloxy;

$C_3$-$C_8$-Cycloalkyl wie Cyclopropyl, Cyclobutyl, Cyclopentyl, Cyclohexyl, Cycloheptyl und Cyclooctyl; vorzugsweise Cycloheptyl, insbesondere Cyclopropyl, Cyclopentyl und Cyclohexyl;

$C_5$-$C_8$-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl; vorzugsweise Cyclopent-3-enyl und Cyclohept-3-enyl, insbesondere Cyclopent-1, Cyclopent-2, Cyclohex-1-enyl und Cyclohex-2-enyl;

oder $C_5$-$C_8$-Cycloalkdienyl wie Cyclopenta-1,3-dien-1-yl, Cyclopenta-1,3-dien-2-yl, Cyclopenta-1,3-dien-5-yl, Cyclohexa-1,3-dien-1-yl, Cyclohexa-1,3-dien-2-yl, Cyclohexa-1,3-dien-5-yl, Cyclohexa-1,4-dien-1-yl, Cyclohexa-1,4-dien-3-yl, Cyclohepta-1,3-dien-1-yl, Cyclohepta-1,3-dien-2-yl, Cyclohepta-1,3-dien-5-yl, Cyclohepta-1,3-dien-6-yl, Cyclohepta-1,4-dien-1-yl, Cyclohepta-1,4-dien-2-yl, Cyclohepta-1,4-dien-3-yl, Cyclohepta-1,4-dien-6-yl, Cycloocta-1,3-dien-1-yl, Cycloocta-1,3-dien-2-yl, Cycloocta-1,3-dien-5-yl, Cycloocta-1,3-dien-6-yl, Cycloocta-1,4-dien-1-yl, Cycloocta-1,4-dien-2-yl, Cycloocta-1,4-dien-3-yl, Cycloocta-1,4-dien-6-yl, Cycloocta-1,4-dien-7-yl, Cycloocta-1,4-dien-1-yl und Cycloocta-1,4-dien-3-yl, vorzugsweise Cyclopenta-1,3-dien-1-yl und Cyclohexa-1,3-dien-2-yl, insbesondere Cyclopenta-1,3-dien-5-yl und Cyclohexa-1,3-dien-1-yl;

wobei diese cyclischen Reste ein bis fünf Halogenatome wie Fluor, Chlor, Brom und Jod, vorzugsweise Fluor, Chlor und Brom, einen Phenyl-, Phenoxy- oder Phenylthiorest und/oder ein bis drei der folgenden Reste tragen können:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Ethyl, insbesondere Methyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und 2,2,2-Trifluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Ethoxy, 1-Methylethoxy und, 1,1-Dimethylethoxy, insbesondere Methoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Fluormethyloxy und Difluormethyloxy, insbesondere Trifluormethyloxy und 2,2-Difluorethyloxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Ethylthio, 1-Methylethylthio und 1,1-Dimethylethylthio, insbesondere Methylthio;

oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise Trichlormethylthio und Fluormethylthio, insbesondere Difluormethylthio und 2,2-Difluorethylthio;

$C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-Pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, vorzugsweise 1-Methyl-1-propenyl, 1-Methyl-1-butenyl, 1-Ethyl-1-propenyl, 1-Hexenyl, 2-Hexenyl, 1-Ethyl-1-butenyl und 1,1,2-Trimethyl-2-propenyl, insbesondere Ethenyl, 2-Propenyl und 1-Methyl-2-propenyl;

oder $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, vorzugsweise 2-Butinyl, 1-Methyl-2-propinyl und 2,2-Dimethyl-3-butinyl,insbesondere Ethinyl und 1-Propinyl;

wobei diese Reste ein bis fünf Halogenatome wie Fluor, Chlor, Brom und Jod, vorzugsweise Brom insbesondere Fluor und Chlor, einen Phenyl-, Phenoxy- oder Phenylthiorest und/oder ein bis drei der folgenden Reste tragen können:

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise 1-Methylethoxy, insbesondere Methoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise 2,2-Difluorethyloxy, insbesondere Difluormethyloxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise 1-Methylethylthio, insbesondere Methylthio;

oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise Trichlormethylthio und 2,2-Difluorethylthio, insbesondere Difluormethylthio;

Amino, welches eine $C_1$-$C_6$-Alkylcarbonylgruppe wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropyl-carbonyl, vorzugsweise Ethylcarbonyl und 1-Methylethyl-carbonyl, insbesondere Methylcarbonyl und 1,1-Dimethylethylcarbonyl;
eine $C_1$-$C_6$-Alkoxycarbonylgruppe wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise 1,1-Dimethylethoxycarbonyl, insbesondere Ethoxycarbonyl;
eine $C_1$-$C_6$-Alkylaminocarbonylgruppe wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methyl-propylaminocarbonyl und 1-Ethyl-2-methylpropylaminocarbonyl, vorzugsweise Ethylaminocarbonyl und 1-Methylethylaminocarbonyl, insbesondere Methylaminocarbonyl;
oder eine Di-$C_1$-$C_6$-alkylaminocarbonylgruppe, besonders Di-$C_1$-$C_4$-alkylaminocarbonyl wie N,N-Dimethylaminocarbonyl, N,N-Diethylaminocarbonyl, N,N-Dipropylaminocarbonyl, N,N-Di-(1-methylethyl)-aminocarbonyl, N,N-Dibutylaminocarbonyl, N,N,Di-(1-methylpropyl)aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propyl- aminocarbonyl, N-Methyl-N-(1-methylethyl)aminocarbonyl, N-Butyl-N-methylaminocarbonyl, N-Methyl-N-(1-methylpropyl)amino-carbonyl, N-Methyl-N-(2-methylpropyl)aminocarbonyl, N-(1,1-Dimethylethyl)-N-methylaminocarbonyl, N-Ethyl-N-propyl-aminocarbonyl, N-Ethyl-N(1-methylethyl)aminocarbonyl, N-Butyl-N-ethylaminocarbonyl, N-Ethyl-N-(1-methylpropyl)amino- carbonyl, N-Ethyl-N-(2-methylpropyl)-aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methyl-propyl)-N-propylaminocarbonyl, M-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)-aminocarbonyl, N-Butyl-N-(2-methylpropyl)-aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl und M-(1,1-Dimethylethyl)-N-(2-methylpropyl)aminocarbonyl, vorzugsweise N,N-Diethylaminocarbonyl,N-Ethyl-N-methylaminocarbonyl und N-Methyl-N-(2-methylpropyl)aminocarbonyl, insbesondere N,N-Dimethylaminocarbonyl;
und/oder ein oder zwei $C_1$-$C_6$-Alkylgruppen wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl, tragen kann;
$C_1$-$C_6$-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Ethylcarbonyl, 1-Methylethyl-carbonyl, insbesondere Methylcarbonyl und 1,1-Dimethylethylcarbonyl;
$C_1$-$C_6$-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl,

Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethyl- propoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyl-oxycarbonyl, 1-Ethyl-1-methylpropyloxycarbonyl und 1-Ethyl-2-methylpropyloxycarbonyl, vorzugsweise 1,1-Dimethylethoxycarbonyl, insbesondere Ethoxycarbonyl;

$C_1$-$C_6$-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methyl-propylaminocarbonyl und 1-Ethyl-2-methylpropylaminocarbonyl, vorzugsweise Ethylaminocarbonyl und 1-Methylethylaminocarbonyl, insbesondere Methylaminocarbonyl;

Di-$C_1$-$C_6$-alkylaminocarbonyl, besonders Di-$C_1$-$C_4$-alkylaminocarbonyl wie N,N-Dimethylaminocarbonyl, N,N-Diethylaminocarbonyl, N,N-Dipropylaminocarbonyl,N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)-aminocarbonyl, N-Ethyl-N-methylaminocarbonyl, N-Methyl-N-propyl-aminocarbonyl, N-Methyl-N-(1-methylethyl)aminocarbonyl, N-Butyl-N-methylaminocarbonyl, N-Methyl-N-(1-methylpropyl)-aminocarbonyl, N-Methyl-N-(2-methylpropyl)aminocarbonyl, N-(1,1-Dimethylethyl)-N-methylaminocarbonyl, N-Ethyl-N-propyl-aminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methyl-propyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)-aminocarbonyl, N-(1-Methylethyl)-N-(2-methylpropyl)aminocarbonyl, N-(1,1-Di-methylethyl)-N-(1-methylethyl)aminocarbonyl, N-Butyl-N-(1-methylpropyl)aminocarbonyl, N-Butyl-N-(2-methylpropyl)-aminocarbonyl, N-Butyl-N-(1,1-dimethylethyl)aminocarbonyl, N-(1-Methylpropyl)-N-(2-methyl-propyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise N-Ethyl-N-methylaminocarbonyl und N-Methyl-N-(2-methylpropyl)aminocarbonyl, insbesondere N,N-Dimethylaminocarbonyl und N,N-Diethylaminocarbonyl;

Benzyloxycarbonyl, Benzoyl, Phenyl-$C_1$-$C_4$-alkyl wie Phenylmethyl, 1-Phenylethyl, 2-Phenylethyl, 1-Phenylpropyl, 2-Phenylpropyl, 3-Phenylpropyl, 1-Phenyl-1-methylethyl, 2-Phenyl-1-methylethyl, 1-Phenylbutyl, 2-Phenylbutyl, 3-Phenylbutyl, 4-Phenylbutyl, 1-Phenyl-1-methylpropyl, 1-Phenyl-2-methylpropyl, 2-Phenyl-1-methylpropyl, 2-Phenyl-2-methylpropyl, 3-Phenyl-1-methylpropyl, 3-Phenyl-2-methylpropyl und 1,1-Dimethyl-2-phenylethyl, vorzugsweise 2-Phenylethyl, insbesondere Phenylmethyl;

Phenyl-$C_1$-$C_4$-alkoxy wie Phenylmethoxy, 1-Phenylethoxy, 2-Phenylethoxy, 1-Phenylpropyloxy, 2-Phenylpropyloxy, 3-Phenylpropyloxy, 1-Phenyl-1-methylethoxy, 2-Phenyl-1-methylethoxy, 1-Phenylbutyloxy, 2-Phenylbutyloxy, 3-Phenylbutyloxy, 4-Phenylbutyloxy, 1-Phenyl-1-methoxypropyloxy, 1-Phenyl-2-methylpropyloxy, 2-Phenyl-1-methylpropyloxy, 2-Phenyl-2-methylpropyloxy, 3-Phenyl-1-methylpropyloxy, 3-Phenyl-2-methylpropyloxy und 1,1-Dimethyl-2-phenylethoxy, vorzugsweise 2-Phenylethoxy und 1-Phenyl-1-methylethoxy, insbesondere Phenylmethoxy;

oder Phenoxy-$C_1$-$C_4$-alkyl wie Phenoxymethyl, 1-Phenoxyethyl, 2-Phenoxyethyl, 1-Phenoxypropyl, 2-Phenoxypropyl, 3-Phenoxypropyl, 1-Phenoxy-1-methylethyl, 2-Phenoxy-1-methylethyl, 1-Phenoxybutyl, 2-Phenoxybutyl, 3-Phenoxybutyl, 4-Phenoxybutyl, 1-Phenoxy-1-methylpropyl, 1-Phenoxy-2-methylpropyl, 2-Phenoxy-1-methylpropyl, 2-Phenoxy-2-methylpropyl, 3-Phenoxy-1-methylpropyl, 3-Phenoxy-2-methylpropyl und 1,1-Dimethyl-2-phenoxyethyl, vorzugsweise 1-Phenoxy-1-methylethyl, insbesondere Phenoxymethyl;

wobei die aromatischen Reste ein bis fünf Halogenatome wie Fluor, Chlor, Brom und Jod, vorzugsweise Brom, insbesondere Fluor und Chlor und/oder ein bis drei der folgenden Reste tragen können:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Ethyl, 1-Methylethyl und 1,1-Dimethylethyl, insbesondere Methyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl,

2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und 2,2,2-Trifluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Ethoxy, 1-Methylethoxy und 1,1-Dimethylethoxy, insbesondere Methoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Fluormethyloxy und Difluormethyloxy, insbesondere Trifluormethyloxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Ethylthio, 1-Methylethylthio und 1,1-Dimethylethylthio, insbesondere Methylthio;

oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise Trichlormethylthio, Fluormethylthio, insbesondere Difluormethylthio und 2,2-Difluorethylthio;

oder $R^1$ gemeinsam mit einem der Reste $R^2$ in ortho-Position

eine 1,3-Butadien-1,4-diylgruppe, wobei dieser Rest ein bis vier Halogenatome wie Fluor, Chlor, Brom und Jod, vorzugsweise Brom, insbesondere Fluor und Chlor und/oder ein oder zwei der folgenden Reste tragen kann:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Ethyl, 1-Methylethyl und 1,1-Dimethylethyl, insbesondere Methyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und 2,2,2-Trifluorethyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Ethoxy, 1-Methylethoxy und 1,1-Dimethylethoxy, insbesondere Methoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Fluormethyloxy und Difluormethyloxy, insbesondere Trifluormethyloxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Ethylthio, 1-Methylethylthio und 1,1-Dimethylethylthio, insbesondere Methylthio;

oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise Trichlormethylthio, Fluormethylthio, insbesondere Difluormethylthio und 2,2-Difluorethylthio;

oder eine drei- oder viergliedrige Kette aus Kohlenstoffgliedern und einem Sauerstoffglied wie Oxyethyl, Methyloxymethyl, Oxypropyl und Methyloxyethyl, vorzugsweise Oxyethyl, wobei diese Kette ein bis drei $C_1$-$C_4$-Alkylgruppen wie Methyl, Ethyl, Propyl, 1-Methylethyl, Butyl, 1-Methylpropyl, 2-Methylpropyl und 1,1-Dimethylethyl, vorzugsweise Ethyl, insbesondere Methyl, tragen kann;

$R^2$

Halogen wie Fluor, Chlor, Brom und Jod, vorzugsweise Brom, insbesondere Fluor und Chlor;

$C_1$-$C_{10}$-Alkyl, besonders $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl;
$C_1$-$C_6$-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, vorzugsweise Ethyloxy, 1-Methylethyloxy und 2,2-Dimethylbutyloxy, insbesondere Methyloxy und 2-Methylpropyloxy, 1,1-Dimethylethyloxy;
$C_2$-$C_6$-Alkenyloxy wie Ethenyloxy, 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1- butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Di-methyl-3-butenyloxy, 1,2-Dimethyl-1-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy und 1-Ethyl-2-methyl-2-propenyloxy, vorzugsweise Ethenyloxy, 1-Methylethenyloxy, 3-Methyl-2-butenyloxy und 3-Methyl-3-butenyloxy, insbesondere 2-Propenyloxy und 1-Methyl-2-propenyloxy;
$C_2$-$C_6$-Alkinyloxy wie Ethinyloxy, 1-Propinyloxy, 2-Propinyloxy, 1-Butinyloxy, 2-Butinyloxy, 3-Butinyloxy, 1-Methyl-2-propinyloxy, 1-Pentinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 3-Methyl-1-butinyloxy, 1,1-Dimethyl-2-propinyloxy, 1-Ethyl-2-propinyloxy, 1-Hexinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-1-pentinyloxy, 3-Methyl-4- pentinyloxy, 4-Methyl-1-pentinyloxy, 4-Methyl-2-pentinyloxy, 1,1-Dimethyl-2-butinyloxy, 1,1-Dimethyl-3-butinyloxy, 1,2-Di-methyl-3-butinyloxy, 2,2-Dimethyl-3-butinyloxy, 3,3-Dimethyl-1-butinyloxy, 1-Ethyl-2-butinyloxy, 1-Ethyl-3-butinyloxy, 2-Ethyl-3-butinyloxy und 1-Ethyl-1-methyl-2-propinyloxy;
oder $C_5$-$C_{10}$-Alkdienyloxy wie 3,7-Diemthyl-2,6-octadienyloxy, 3,7-Dimethyl-3,7-octadienyloxy, 3,7-Dimethyl-2,7-octadienyloxy und 3,7-Dimethyl-3,6-octydienyloxy;
wobei diese Reste ein bis fünf Halogenatome wie Fluor, Chlor, Brom und Jod, vorzugsweise Brom, insbesondere Fluor oder Chlor, einen Phenyl-, Phenoxy- oder Phenylthiorest und/oder ein bis drei der folgenden Reste tragen können:
$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Ethoxy, 1-Methylethoxy und 1,1-Dimethylethoxy, insbesondere Methoxy;
$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Fluormethyloxy und Difluormethyloxy, insbesondere Trifluormethyloxy;
$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Ethylthio, 1-Methylethylthio und 1,1-Dimethylethylthio, insbesondere Methylthio;
oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio

und Pentafluorethylthio, vorzugsweise Trichlormethylthio, Fluormethylthio, insbesondere Difluormethylthio, 2,2-Difluorethylthio;
Benzyloxy oder Benzylthio, wobei die aromatischen Reste ein bis fünf Halogenatome wie Fluor, Chlor, Brom und Jod, vorzugsweise Brom, insbesondere Fluor und Chlor, einen Phenyl-, Phenoxy- oder Phenylthiorest und/oder ein bis drei der folgenden Reste tragen können:
$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Ethyl, 1-Methylethyl und 1,1-Dimethylethyl, insbesondere Methyl;
$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Difluormethyl und 2,2,2-Trifluorethyl, insbesondere Trifluormethyl;
$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Ethoxy, 1-Methylethoxy und 1,1-Dimethylethoxy, insbesondere Methoxy;
$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Fluormethyloxy und Difluormethyloxy, insbesondere Trifluormethyloxy;
$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Ethylthio, 1-Methylethylthio, 1,1-Dimethylethylthio, insbesondere Methylthio;
oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise Trichlormethylthio und Fluormethylthio, insbesondere Difluormethylthio, 2,2-Difluorethylthio, zur Bekämpfung von Schädlingen.

Es versteht sich, daß die Verbindungen der Formel I in Abhängigkeit von den eingesetzten Ausgangsstoffen und den Umsetzungbedingungen sowohl in Form von reinen Strukturisomeren, als auch in Form von Isomerenpaaren (Enantiomere und Diastereomere) bzw. in Form von Isomerengemischen erhalten werden können und als solche als Wirkstoffe Verwendung finden können.

Isomerengemische bzw. Isomerenpaare können in üblicher Weise in die sterisch einheitlichen Komponenten aufgetrennt werden. Die biologische Aktivität ist in Einzelfällen von der sterischen Konfiguration der Verbindungen abhängig.

Im Hinblick auf ihre Verwendung zur Schädlingsbekämpfung sind insbesondere solche alpha-Arylacrylsäurederivate der Formel I bevorzugt, in denen die Substituenten und Indizes folgende Bedeutung haben:
X
Ethenylen;
n
0 oder 1;
m
0, 1, 2 oder 3, wobei die Reste $R^2$ verschieden sein können, wenn m für 2 oder 3 steht;
$R^1$
Cyano;
$C_3$-$C_8$-Cycloalkyl, $C_5$-$C_8$-Cycloalkenyl oder $C_5$-$C_8$-Cycloalkdienyl wie vorstehend im allgemeinen und im besonderen genannt, wobei diese ein bis fünf Halogenatome wie vorstehend im allgemeinen und im besonderen genannt, einen Phenyl-, Phenoxy- oder Phenylthiorest und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio wie vorstehend im allgemeinen und im besonderen genannt;
$C_2$-$C_6$-Alkenyl wie vorstehend im allgemeinen und im besonderen genannt, wobei dieser Rest ein bis fünf Halogenatome wie vorstehend im allgemeinen und im besonderen genannt, einen Phenyl-, Phenoxy- oder Phenylthiorest und/oder ein bis drei der folgenden Reste tragen kann: $C_1$-$C_4$-Alkoxy, $C_1$-$C_4$-Halogenalkoxy, $C_1$-$C_4$-Alkylthio oder $C_1$-$C_4$-Halogenalkylthio wie vorstehend im allgemeinen und im besonderen genannt;
$R^2$
Halogen wie vorstehend im allgemeinen und im besonderen genannt;
$C_1$-$C_4$-Alkyl, $C_1$-$C_4$-Alkoxy, $C_2$-$C_6$-Alkenyloxy oder $C_5$-$C_{10}$-Alkdienyloxy wie vorstehend im allgemeinen und

im besonderen genannt, wobei diese Reste ein bis fünf Halogenatome wie vorstehend im allgemeinen und im besonderen genannt, einen Phenyl-, Phenoxy- oder Phenylthiorest und/oder ein bis drei der folgenden Reste tragen können: $C_1$-$C_4$-Alkoxy, $C_1$-$C_4$-Halogenalkoxy, $C_1$-$C_4$-Alkylthio oder $C_1$-$C_4$-Halogenalkylthio wie vorstehend im allgemeinen und im besonderen genannt.

Beispiele für insbesondere bevorzugte $\alpha$-Arylacrylsäurederivate der allgemeinen Formeln IA bzw. IB sind in den folgenden Tabellen A und B aufgeführt.

Tabelle A

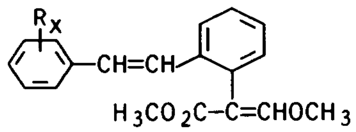

IA

($R = R^1 + R^2$; $x = n + m$)

| $R_x$ |
|---|
| 2-F |
| 3-F |
| 4-F |
| 2,3-$F_2$ |
| 2,4-$F_2$ |
| 2,5-$F_2$ |
| 2,6-$F_2$ |
| 3,4-$F_2$ |
| 3,5-$F_2$ |
| 2-Cl |
| 3-Cl |
| 4-Cl |
| 2,3-$Cl_2$ |
| 2,4-$Cl_2$ |

Tabelle A (Fortsetzung)

| $R_x$ |
|---|
| 2,5-$Cl_2$ |
| 2,6-$Cl_2$ |
| 3,4-$Cl_2$ |
| 3,5-$Cl_2$ |
| 2-Br |
| 3-Br |
| 4-Br |
| 2,3-$Br_2$ |
| 2,4-$Br_2$ |
| 2,5-$Br_2$ |
| 2,6-$Br_2$ |
| 3,4-$Br_2$ |
| 3,5-$Br_2$ |
| 2-Cl, 6-F |
| 3-Cl, 4-F |
| 3-Cl, 2-F |
| 2,3,4-$Cl_3$ |
| 2,3,6-$Cl_3$ |
| 3,5-$Br_2$, 2-$OCH_3$ |
| 3,5-$Br_2$, 2-$OC_2H_5$ |
| 3,5-$Br_2$, 2-O-i-$C_3H_7$ |
| 3-Br, 4-$OCH_3$ |
| 3-Br, 2-$OCH_3$ |
| 3-Br, 4,5-$(OCH_3)_2$ |
| 2-$OCH_3$ |
| 3-$OCH_3$ |
| 4-$OCH_3$ |
| 3,4-$(OCH_3)_2$ |
| 3,5-$(OCH_3)_2$ |
| 3,4,5-$(OCH_3)_3$ |
| 2-$OCH_3$, 3-$OC_2H_5$ |
| 2,4,5-$(OCH_3)_3$ |
| 2-$OC_2H_5$ |
| 3-$OC_2H_5$ |
| 4-$OC_2H_5$ |
| 3-$OC_2H_5$, 4-$OCH_3$ |
| 3-$OC_2H_5$, 4-O-i-$C_3H_7$ |
| 3-$OC_2H_5$, 4-O-t-$C_4H_9$ |

Tabelle A (Fortsetzung)

| $R_x$ |
|---|
| $3,5-(OC_2H_5)_2$ |
| $3-O-i-C_3H_7$ |
| $4-O-i-C_3H_7$ |
| $3-O-t-C_4H_9$ |
| $4-O-t-C_4H_9$ |
| $4-O-i-C_4H_9$ |
| $3-O-i-C_4H_9$ |
| $3,4-(O-t-C_4H_9)_2$ |
| $3,5-(O-t-C_4H_9)_2$, $4-OCH_3$ |
| 3,4-( 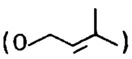 )2 |
| 3,4-( 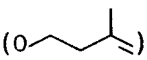 )2 |
| 4- 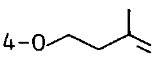 |
| 4- 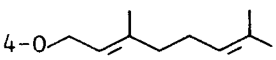 |
| 3,4-( 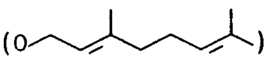 )2 |
| $3-OCH_2C_6H_5$ |
| $4-OCH_2C_6H_5$ |
| $2-OCH_2C_6H_5$, $3-OCH_3$ |
| $3-OCH_2C_6H_5$, $4-OCH_3$ |
| $4-OCH_2C_6H_5$, $3-OCH_3$ |
| $3,4-(OCH_2C_6H_5)_2$ |
| $3-OCHF_2$ |
| $4-OCHF_2$ |
| $2-OCF_2CHF_2$ |
| $3-OCF_2CHF_2$ |
| $4-OCF_2CHF_2$ |
| $3-CF_3$ |
| $4-CF_3$ |
| $2-CH_3$ |
| $3-CH_3$ |
| $4-CH_3$ |
| $2,4-(CH_3)_2$ |
| $3,4-(CH_3)_2$ |
| $3-C_2H_5$ |

Tabelle A (Fortsetzung)

| $R_x$ |
|---|
| 4-$C_2H_5$ |
| 3-t-$C_4H_9$ |
| 4-t-$C_4H_9$ |
| 3-t-$C_4H_9$, 4-$OCH_3$ |
| 3,5-(t-$C_4H_9$)$_2$, 4-$OCH_3$ |
| 3-$CH_3$, 4-$OCH_3$ |
| 3,4-(t-$C_4H_9$)$_2$ |
| 2,3-CH=CH-CH=CH |
| 3,4-CH=CH-CH=CH |
| 3-Cyclopentenyl, 2-$CH_3$ |
| 3-Cyclohexenyl, 2-$CH_3$ |
| 3-Cycloheptenyl, 2-$CH_3$ |
| 3-(2-Norbornen-2-yl), 2-$CH_3$ |
| 3-Cyclohexenyl, 2-$CH_3$, 5-F |
| 3-Cyclopentenyl, 2-$CH_3$, 6-Cl |
| 3-(1,3-Cyclohexadienyl), 2-$CH_3$ |
| 3-Cyclopropyl, 2-$CH_3$ |
| 3-Norbornen-2-yl, 2-$CH_3$ |
| 3-i-$C_3H_7$, 2-$CH_3$ |
| 3-CH($C_2H_5$)$_2$, 2-$CH_3$ |
| 3-CH($CH_3$)(n-$C_3H_7$), 2-$CH_3$ |
| 3-s-$C_4H_9$, 2-$CH_3$ |
| 3-n-$C_4H_9$, 2-$CH_3$ |
| 3-CH(i-$C_3H_7$)$_2$, 2-$CH_3$ |
| 3-CH($CH_3$)(n-$C_3H_7$), 2-$CH_3$ |
| 3-C($CH_3$)=$CH_2$, 2-$CH_3$ |
| 3-C($C_2H_5$)=$CH_2$, 2-$CH_3$ |
| 3-CH=$CH_2$, 2-$CH_3$ |
| 3-C($CH_3$)=$CHCH_3$, 2-$CH_3$ |
| 3-C($CH_2CH_3$)=$CHCH_3$, 2-$CH_3$ |
| 3-$CH_3$, 2-Cl |
| 3-$C_2H_5$, 2-Cl |
| 3-i-$C_3H_7$, |
| 3-t-$C_4H_9$, 2-Cl |
| 3-s-$C_4H_9$, 2-Cl |
| 3-Cyclopentyl, 2-Cl |
| 3-Cyclohexyl, 2-Cl |
| 3-Cycloheptyl, 2-Cl |

Tabelle A (Fortsetzung)

| $R_X$ |
|---|
| 3-Norborn-2-yl, 2-Cl |
| 3-(2-Cyclopentenyl),2-Cl |
| 3-(2-Cyclohexenyl), 2-Cl |
| 3-$C(CH_3)=CHCH_3$, 2-Cl |
| 3-$CH(CH_3)CH=CH_2$, 2-Cl |
| 3-$CH_3$, 2-F |
| 3-$C_2H_5$, 2-F |
| 3-i-$C_3H_7$, 2-F |
| 3-s-$C_4H_9$, 2-F |
| 3-t-$C_4H_9$, 2-F |
| 3-$CH(C_2H_5)_2$, 2-F |
| 3-Cyclopropyl, 2-F |
| 3-Cyclopentyl, 2-F |
| 3-Cyclohexyl, 2-F |
| 3-(2-Cyclohexenyl), 2-F |
| 3-(Norborn-2-yl), 2-F |
| 3-$C(CH_3)=CH_2$, 2-F |
| 3-$CH_3$, 2-Br |
| 3-i-$C_3H_7$, 2-Br |
| 3-t-$C_4H_9$, 2-Br |
| 3-Cyclopropyl, 2-Br |
| 3-Cyclopentyl, 2-Br |
| 3-Cyclohexyl, 2-Br |
| 3-$CH_3$, 2-$OCH_3$ |
| 3-i-$C_3H_7$, 2-$OCH_3$ |
| 3-t-$C_4H_9$, 2-$OCH_3$ |
| 3-Cyclopropyl, 2-$OCH_3$ |
| 3-Cyclopentyl, 2-$OCH_3$ |
| 3-Cyclohexyl, 2-$OCH_3$ |
| 3-CN |
| 4-CN |
| 4-SCN |
| 3-$NO_2$ |
| 4-$NO_2$ |
| 3-$NO_2$, 4-$CH_3$ |
| 3-$NO_2$, 4-Cl |
| 3-$N(CH_3)_2$ |
| 4-$N(CH_3)_2$ |

Tabelle A (Fortsetzung)

| $R_x$ |
|---|
| 3-$NHCOCH_3$ |
| 3-$NHCO_2CH_3$ |
| 4-$NHCO_2CH_3$ |
| 4-$NHCON(CH_3)_2$ |
| 3-$CO_2CH_3$ |
| 3-$CO_2C_2H_5$ |
| 3-$CO_2$-i-$C_3H_7$ |
| 3-$CO_2$-t-$C_4H_9$ |
| 4-$CO_2CH_3$ |
| 4-$CO_2C_2H_5$ |
| 4-$CO_2$-i-$C_3H_7$ |
| 4-$CO_2$-t-$C_4H_9H$ |
| 3-$CO_2CH_2C_6H_5$ |
| 4-$CO_2CH_2C_6H_5$ |
| 3-Benzoyl |
| 4-Benzoyl |
| 4-$CONHCH_3$ |
| 3-$CONHCH_3$ |
| 3-$CON(CH_3)_2$ |
| 3-Phenethyl |
| 4-Phenethyl |
| 3-Phenethyloxy |
| 4-Phenethyloxy |
| 3-Phenoxymethyl |
| 4-Phenoxyethyl |
| 3,4-$OC(CH_3)_2CH_2$ |
| 4-$OCH_2CH(C_2H_5)$(n-$C_4H_9$) |

Tabelle B

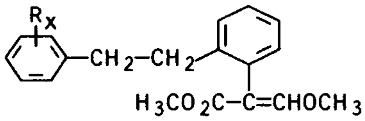

IB

($R = R^1 + R^2$; $x = n + m$)

Tabelle B

| $R_x$ |
|---|
| 2-F, |
| 3-F, |
| 4-F, |
| 2,3-$F_2$ |
| 2,4-$F_2$ |
| 2,5-$F_2$ |
| 2,6-$F_2$ |
| 3,4-$F_2$ |
| 3,5-$F_2$ |
| 2-Cl |
| 3-Cl |
| 4-Cl |
| 2,3-$Cl_2$ |
| 2,4-$Cl_2$ |
| 2,5-$Cl_2$ |
| 2,6-$Cl_2$ |
| 3,4-$Cl_2$ |
| 3,5-$Cl_2$ |
| 2-Br |
| 3-Br |
| 4-Br |
| 2,3-$Br_2$ |
| 2,4-$Br_2$ |
| 2,5-$Br_2$ |
| 2,6-$Br_2$ |
| 3,4-$Br_2$ |
| 3,5-$Br_2$ |
| 2-Cl, 6-F |
| 3-Cl, 4-F |
| 3-Cl, 2-F |
| 2,3,4-$Cl_3$ |
| 2,3,6-$Cl_3$ |

Tabelle B (Fortsetzung)

| $R_x$ |
|---|
| 3,5-$Br_2$, 2-$OCH_3$ |
| 3,5-$Br_2$, 2-$OC_2H_5$ |
| 3,5-$Br_2$, 2-O-i-$C_3H_7$ |
| 3-Br, 4-$OCH_3$ |
| 3-Br, 2-$OCH_3$ |
| 3-Br, 4,5-$(OCH_3)_2$ |
| 2-$OCH_3$ |
| 3-$OCH_3$ |
| 4-$OCH_3$ |
| 3,4-$(OCH_3)_2$ |
| 3,5-$(OCH_3)_2$ |
| 3,4,5-$(OCH_3)_3$ |
| 2-$OCH_3$, 3-$OC_2H_5$ |
| 2,4,5-$(OCH_3)_3$ |
| 2-$OC_2H_5$ |
| 3-$OC_2H_5$ |
| 4-$OC_2H_5$ |
| 3-$OC_2H_5$, 4-$OCH_3$ |
| 3-$OC_2H_5$, 4-O-i-$C_3H_7$ |
| 3-$OC_2H_5$, 4-O-t-$C_4H_9$ |
| 3,5-$(OC_2H_5)_2$ |
| 3-O-i-$C_3H_7$ |
| 4-O-i-$C_3H_7$ |
| 3-O-t-$C_4H_9$ |
| 4-O-t-$C_4H_9$ |
| 4-O-i-$C_4H_9$ |
| 3-O-i-$C_4H_9$ |
| 3,4-$(O\text{-}t\text{-}C_4H_9)_2$ |
| 3,5-$(O\text{-}t\text{-}C_4H_9)_2$, 4-$OCH_3$ |
| 3-$OCHF_2$ |
| 4-$OCHF_2$ |
| 2-$OCF_2CHF_2$ |
| 3-$OCF_2CHF_2$ |
| 4-$OCF_2CHF_2$ |
| 3-$CF_3$ |
| 4-$CF_3$ |
| 2-$CH_3$ |
| 3-$CH_3$ |

Tabelle B (Fortsetzung)

| $R_x$ |
|---|
| 4-$CH_3$ |
| 2,4-$(CH_3)_2$ |
| 3,4-$(CH_3)_2$ |
| 3-$C_2H_5$ |
| 4-$C_2H_5$ |
| 3-t-$C_4H_9$ |
| 4-t-$C_4H_9$ |
| 3-t-$C_4H_9$, 4-$OCH_3$ |
| 3,5-(t-$C_4H_9)_2$, 4-$OCH_3$ |
| 3-$CH_3$, 4-$OCH_3$ |
| 3,4-(t-$C_4H_9)_2$ |
| 2,3-CH=CH-CH=CH |
| 3,4-CH=CH-CH=CH |
| 3-Cyclopropyl, 2-$CH_3$ |
| 3-Norbornen-2-yl, 2-$CH_3$ |
| 3-i-$C_3H_7$, 2-$CH_3$ |
| 3-CH($C_2H_5)_2$, 2-$CH_3$ |
| 3-CH($CH_3$)(n-$C_3H_7$), 2-$CH_3$ |
| 3-s-$C_4H_9$, 2-$CH_3$ |
| 3-n-$C_4H_9$, 2-$CH_3$ |
| 3-CH(i-$C_3H_7)_2$, 2-$CH_3$ |
| 3-CH($CH_3$)(n-$C_3H_7$), 2-$CH_3$ |
| 3-$CH_3$, 2-Cl |
| 3-$C_2H_5$, 2-Cl |
| 3-i-$C_3H_7$, 2-Cl |
| 3-t-$C_4H_9$, 2-Cl |
| 3-s-$C_4H_9$, 2-Cl |
| 3-Cyclopentyl, 2-Cl |
| 3-Cyclohexyl, 2-Cl |
| 3-Cycloheptyl, 2-Cl |
| 3-Norborn-2-yl, 2-Cl |
| 3-$CH_3$, 2-F |
| 3-$C_2H_5$, 2-F |
| 3-i-$C_3H_7$, 2-F |
| 3-s-$C_4H_9$, 2-F |
| 3-t-$C_4H_9$, 2-F |
| 3-CH($C_2H_5)_2$, 2-F |
| 3-Cyclopropyl, 2-F |

Tabelle B (Fortsetzung)

| $R_x$ |
|---|
| 3-Cyclopentyl, 2-F |
| 3-Cyclohexyl, 2-F |
| 3-(Norborn-2-yl), 2-F |
| 3-$CH_3$, 2-Br |
| 3-i-$C_3H_7$, 2-Br |
| 3-t-$C_4H_9$, 2-Br |
| 3-Cyclopropyl, 2-Br |
| 3-Cyclopentyl, 2-Br |
| 3-Cyclohexyl, 2-Br |
| 3-$CH_3$, 2-$OCH_3$ |
| 3-i-$C_3H_7$, 2-$OCH_3$ |
| 3-t-$C_4H_9$, 2-$OCH_3$ |
| 3-Cyclopropyl, 2-$OCH_3$ |
| 3-Cyclopentyl, 2-$OCH_3$ |
| 3-Cyclohexyl, 2-$OCH_3$ |
| 4-SCN |
| 3-$NO_2$ |
| 4-$NO_2$ |
| 3-$NO_2$, 4-$CH_3$ |
| 3-$NO_2$, 4-Cl |
| 3-$N(CH_3)_2$ |
| 4-$N(CH_3)_2$ |
| 3-$NHCOCH_3$ |
| 3-$NHCO_2CH_3$ |
| 4-$NHCO_2CH_3$ |
| 4-$NHCON(CH_3)_2$ |
| 3-$CO_2CH_3$ |
| 3-$CO_2C_2H_5$ |
| 3-$CO_2$-i-$C_3H_7$ |
| 3-$CO_2$-t-$C_4H_9$ |
| 4-$CO_2CH_3$ |
| 4-$CO_2C_2H_5$ |
| 4-$CO_2$-i-$C_3H_7$ |
| 4-$CO_2$-t-$C_4H_9$ |
| 3-Benzoyl |
| 4-Benzoyl |
| 4-$CONHCH_3$ |
| 3-$CONHCH_3$ |

Tabelle B (Fortsetzung)

| $R_x$ |
|---|
| 3-$CON(CH_3)_2$ |
| 3-Phenethyl |
| 4-Phenethyl |
| 3-Phenethyl |
| 4-Phenethyl |
| 3-Phenethyloxy |
| 4-Phenethyloxy |
| 3-Phenoxymethyl |
| 4-Phenoxyethyl |
| 3,4-$OC(CH_3)_2CH_2$ |
| 4-$OCH_2CH(C_2H_5)(n\text{-}C_4H_9)$ |

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae,

Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl

bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 1.001 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 2.013 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 1.005 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 1.036 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 1.009 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 2.002 mit 10 Gew.-Teilen N-Methyl-$\alpha$-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 1.004 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 1.002 werden mit 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 3, vorzugsweise 0,05 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

2-(β-Methoxy-α-methoxycarbonylvinyl)-3'-chlor-4'-fluor-stilben (Wirkstoffbeispiel 1.015)

Eine Suspension aus 0,94 g Natriumhydrid in 100 ml abs. Tetrahydrofuran wurde bei 25 ° C mit einer Lösung aus 9,4 g 2-(β-Methoxy-α-methoxycarbonylvinyl)-benzylphosphonsäuredimethylester, 5,1 g 3-Chlor-4-fluorbenzaldehyd und 100 ml abs. Tetrahydrofuran versetzt. Nach 12 h wurde die Reaktionsmischung in Eiswasser gegeben. Nach der Extraktion (tert.-Butylmethylether) und der chromatographischen Reinigung (Kieselgel; Toluol/Essigsäureethylester 9:1) erhielt man 6,3 g des Produkts als gelbes Öl (E/Z-Verhältnis 9:1)

α-[2-(3'-Chlor-4'-fluor)-phenylethylphenyl]-β-methoxyacrylsäuremethylester (Wirkstoffbeispiel 2.022)

2 g 2-(β-Methoxy-α-methoxycarbonylvinyl)-3'-chlor-4'-fluor-stilben wurden bei 25 ° C in 100 ml Tetrahydrofuran in Gegenwart von 1 g Pd/C (10 %ig) mit 0,3 bar Wasserstoffüberdruck hydriert. Nach 3 h wurde der Katalysator und das Lösungsmittels entfernt. Man erhielt 1,25 g des Produkts; Fp. 70 bis 75 ° C.

Tabelle 1

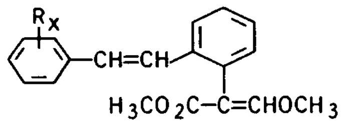

IA

($R = R^1 + R^2$;
$x = n + m$)

| Nr. | $R_x$ | phys. Daten (Fp [°C]; IR [$cm^{-1}$]) |
|---|---|---|
| 1.001 | 2-F | 69- 70 |
| 1.002 | 3-F | 103-105 |
| 1.003 | 4-F | 137-140 |
| 1.004 | 2,4-$F_2$ | Öl |
| 1.005 | 3,4-$F_2$ | 107-110 |
| 1.006 | 2-Cl | 147-148 |
| 1.007 | 3-Cl | 110-111 |
| 1.008 | 4-Cl | 113-115 |
| 1.009 | 2,4-$Cl_2$ | 134-136 |
| 1.010 | 2,6-$Cl_2$ | 127-128 |
| 1.011 | 3,4-$Cl_2$ | 96- 98 |
| 1.012 | 3,5-$Cl_2$ | 144-147 |
| 1.013 | 4-Br | 138-139 |
| 1.014 | 2-Cl, 6-F | 139-140 |
| 1.015 | 3-Cl, 4-F | 1708,1632,1501,1258,1129 |
| 1.016 | 3-Br, 4-$OCH_3$ | 86- 88 |
| 1.017 | 3-Br, 2-$OCH_3$ | 122 |
| 1.018 | 2-$OCH_3$ | 60- 62 |
| 1.019 | 3-$OCH_3$ | 102-103 |
| 1.020 | 4-$OCH_3$ | 125-127 |
| 1.021 | 3,4,5-$(OCH_3)_3$ | 118-121 |
| 1.022 | 3-$OC_2H_5$ | 88- 92 |
| 1.023 | 3-$OC_2H_5$, 4-$OCH_3$ | 113-117 |
| 1.024 | 4-O-t-$C_4H_9$ | 136-137 |
| 1.025 | 4-O-i-$C_4H_9$ | 98 |
| 1.026 | 3,4-(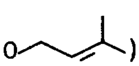)$_2$ | 78 |
| 1.027 | 3,4-(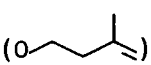)$_2$ | 2940,1710,1630,1510,1260,1130 |
| 1.028 | 4-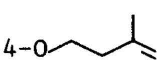 | 84 |
| 1.029 | 4-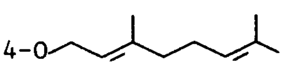 | 83 |
| 1.030 | 3,4-(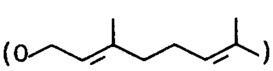)$_2$ | 67 |

Tabelle 1 (Fortsetzung)

| Nr. | $R_x$ | phys. Daten (Fp [°C]; IR [$cm^{-1}$]) |
|---|---|---|
| 1.031 | 3-$OCH_2C_6H_5$ | 67- 70 |
| 1.032 | 3-$OCH_2C_6H_5$, 4-$OCH_3$ | 142-147 |
| 1.033 | 4-$OCH_2C_6H_5$, 3-$OCH_3$ | 117-122 |
| 1.034 | 3,4-(O $CH_2C_6H_5)_2$ | 109-111 |
| 1.035 | 2-$OCF_2CHF_2$ | 1708,1634,1259,1192,1127 |
| 1.036 | 3-$OCF_2CHF_2$ | 1708,1635,1258,1196,1125 |
| 1.037 | 4-$OCF_2CHF_2$ | 104-106 |
| 1.038 | 3-$CF_3$ | 75- 77 |
| 1.039 | 4-$CF_3$ | 124-125 |
| 1.040 | 2-$CH_3$ | 156-158 |
| 1.041 | 3-$CH_3$ | 95- 97 |
| 1.042 | 4-$CH_3$ | 138-139 |
| 1.043 | 2,4-$(CH_3)_2$ | 115-116 |
| 1.044 | 3-t-$C_4H_9$ | Öl |
| 1.045 | 4-t-$C_4H_9$ | Öl |
| 1.046 | 3-t-$C_4H_9$, 4-$OCH_3$ | 140-142 |
| 1.047 | 3,5-(t-$C_4H_9)_2$, 4-$OCH_3$ | 95-102 |
| 1.048 | 2,3-CH=CH-CH=CH | Harz |
| 1.049 | 3,4-CH=CH-CH=CH | 158-159 |
| 1.050 | 3-C($CH_2CH_3$)=$CHCH_3$, 2-$CH_3$ | 1711,1633,1434,1255,1128 |
| 1.051 | 3-i-$C_3H_7$, 2-Cl | 1709,1633,1256,1128 |
| 1.052 | 3-Cyclopentyl, 2-Cl | 1710,1634,1256,1128 |
| 1.053 | 3-$NO_2$, 4-Cl | 168-169 |
| 1.054 | 3,4-OC$(CH_3)_2CH_2$ | 1709,1631,1499,1434,1256,1128 |
| 1.055 | 4-$OCH_2CH(C_2H_5)(n-C_4H_9)$ | 88 |

Tabelle 2

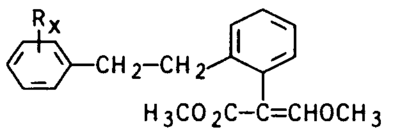

IB

(R = $R^1$ + $R^2$; x = n + m)

| Nr. | $R_x$ | phys. Daten (Fp [°C]; IR [$cm^{-1}$]) |
|---|---|---|
| 2.001 | 2-F | 48- 50 |
| 2.002 | 3-F | 46- 48 |
| 2.003 | 4-F | 51- 52 |
| 2.004 | 2-Cl | 69- 72 |
| 2.005 | 3-Cl | 45- 48 |
| 2.006 | 2,6-$Cl_2$ | 119-121 |
| 2.007 | 3,4-$Cl_2$ | 87- 89 |
| 2.008 | 2-$OCH_3$ | 72- 75 |
| 2.009 | 3-$OCH_3$ | 26- 30 |
| 2.010 | 3-$OC_2H_5$ | 1708,1634,1443,1255,1126 |
| 2.011 | 2-$OCF_2CHF_2$ | 1707,1635,1492,1195,1126 |
| 2.012 | 4-$OCF_2CHF_2$ | 1707,1635,1508,1190,1125 |
| 2.013 | 3-$CF_3$ | 48- 50 |
| 2.014 | 4-$CF_3$ | 72- 75 |
| 2.015 | 4-$CH_3$ | 46- 49 |
| 2.016 | 3-t-$C_4H_9$ | 1709,1634,1254,1127 |
| 2.017 | 3-t-$C_4H_9$, 4-$OCH_3$ | 1709,1634,1496,1255,1126 |
| 2.018 | 3,5-(t-$C_4H_9$)$_2$, 4-$OCH_3$ | 2953,1710,1635,1257,1126 |
| 2.019 | 3,4-CH=CH-CH=CH | 92- 93 |
| 2.020 | 3-$CH_3$, 2-Cl | 56- 58 |
| 2.021 | 4-N($CH_3$)$_2$ | 76- 78 |
| 2.022 | 3-Cl, 4-F | 70- 75 |
| 2.023 | 3,4,5-$Cl_3$ | Öl |
| 2.024 | 2,3-CH=CH-CH=CH | Öl |

Anwendungsbeispiele

Die insektizide Wirkung der Verbindungen der allgemeinen Formel I ließ sich durch folgende Versuche zeigen:

Die Wirkstoffe wurden

a) als 0,1 %-ige Lösung in Aceton oder

b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

A. Musca domestica (Stubenfliege), Zuchtversuch

25 ml einer trockenen Futtermischung (1 kg Kleie, 250 g Hefepulver, 35 g Fischmehl) wurde mit dem Wirkstoff und 25 ml einer Milch-Zucker Lösung (1 l Milch, 42 g Zucker) vermischt und anschließend mit 20 Larven des 1. Entwicklungsstadiums (L1) belegt.

Nach dem Schlüpfen der Larven in einem Kontrollexperiment wurde die Mortalität bestimmt.

In diesem Test zeigten die Verbindungen 1.001, 1.002, 1.003, 1.004, 1.005, 1.006, 1.007, 1.008, 1.009, 1.011, 1.015, 1.016, 1.020, 1.023, 1.024, 1.034, 1.036, 1.037, 1.039, 1.041, 1.042, 1.043, 1.044, 1.045 und 2.012 Wirkschwellen von 2 bis 100 ppm.

B. Plutella maculipennis (Kohlschabe), Fraßhemmung

Junge Kohlrabiblätter wurden mit der wäßrigen Wirkstoffaufbereitung benetzt und anschließend auf einen angefeuchteten Filter gelegt. Die präparierten Blätter wurden anschließend mit jeweils 10 Raupen des 4. Entwicklungsstadiums belegt.

Nach 48 h wurde die Fraßhemmung bestimmt.

In diesem Test zeigten die Verbindungen 1.001, 1.002, 1.003, 1.004, 1.005, 1.006, 1.007, 1.009, 1.011, 1.015, 1.016, 1.023, 1.031, 1.034, 1.036, 1.041, 1.043, 1.044, 1.049 und 2.004 Wirkschwellen von 40 bis 1000 ppm.

C. Tetranychus telarius (Rote Spinne), Kontaktwirkung

Stark mit Milben befallene getopfte Buschbohnen, die das zweite Folgeblattpaar zeigten, wurden mit wässrigen Wirkstoffaufbereitung tropfnaß gespritzt.

Nach 5 Tagen im Gewächshaus wurde der Bekämpfungserfolg mittels Binokular bestimmt.

In diesem Test zeigten die Verbindungen 1.001, 1.002, 1.004, 1.005, 1.009, 1.015, 1.034, 1.035, 1.039, 1.041, 1.044, 1.045, 1.050, 1.051, 1.052, 1.054, 2.002, 2.005, 2.013, 2.016, 2.017 und 2.020 Wirkschwellen von 100 bis 1000 ppm.

## Patentansprüche

**1.** Verwendung von alpha-Arylacrylsäurederivaten der allgemeinen Formel I,

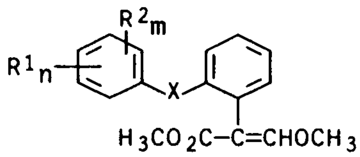

I

in der die Substituenten und Indizes folgende Bedeutung haben:

X
Ethylen oder Ethenylen;

n
0 oder 1;

m
0, 1, 2, 3 oder 4, wobei die Reste $R^2$ verschieden sein können, wenn m für 2, 3 oder 4 steht;

$R^1$
Cyano; Cyanato; Thiocyanato; Nitro; Hydroxy; Carboxyl;

$C_1$-$C_4$-Halogenalkyl; $C_1$-$C_4$-Halogenalkoxy;

$C_3$-$C_8$-Cycloalkyl, $C_5$-$C_8$-Cycloalkenyl oder $C_5$-$C_8$-Cycloalkdienyl, wobei diese Reste ein bis fünf Halogenatome, einen Phenyl-, Phenoxy- oder Phenylthiorest und/oder ein bis drei der folgenden Reste

tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

$C_2$-$C_6$-Alkenyl oder $C_2$-$C_6$-Alkinyl, wobei diese Reste ein bis fünf Halogenatome, einen Phenyl-, Phenoxy- oder Phenylthiorest und/oder ein bis drei der folgenden Reste tragen können: $C_1$-$C_4$-Alkoxy, $C_1$-$C_4$-Halogenalkoxy, $C_1$-$C_4$-Alkylthio oder $C_1$-$C_4$-Halogenalkylthio;

Amino, welches eine $C_1$-$C_6$-Alkylcarbonyl-, $C_1$-$C_6$-Alkoxycarbonyl-, $C_1$-$C_6$-Alkylaminocarbonyl- oder Di-$C_1$-$C_6$-alkylaminocarbonylgruppe und/oder ein oder zwei $C_1$-$C_6$-Alkylgruppen tragen kann;

$C_1$-$C_6$-Alkylcarbonyl; $C_1$-$C_6$-Alkoxycarbonyl; $C_1$-$C_6$-Alkylaminocarbonyl; Di-$C_1$-$C_6$-alkylaminocarbonyl;

Benzyloxycarbonyl, Benzoyl, Phenyl-$C_1$-$C_4$-alkyl, Phenyl-$C_1$-$C_4$-alkoxy oder Phenoxy-$C_1$-$C_4$-alkyl, wobei die aromatischen Reste ein bis fünf Halogenatome und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

oder $R^1$ gemeinsam mit einem der Reste $R^2$ in ortho-Position

eine 1,3-Butadien-1,4-diylgruppe, wobei dieser Rest ein bis vier Halogenatome und/oder ein oder zwei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio,

oder eine drei- oder viergliedrige Kette aus Kohlenstoffgliedern und einem Sauerstoffglied, wobei diese Kette ein bis drei $C_1$-$C_4$-Alkylgruppen tragen kann;

$R^2$
Halogen;

$C_1$-$C_{10}$-Alkyl, $C_1$-$C_6$-Alkoxy, $C_2$-$C_6$-Alkenyloxy, $C_2$-$C_6$-Alkinyloxy oder $C_5$-$C_{10}$-Alkdienyloxy, wobei diese Reste ein bis fünf Halogenatome, einen Phenyl-, Phenoxy- oder Phenylthiorest und/oder ein bis drei der folgenden Reste tragen können: $C_1$-$C_4$-Alkoxy, $C_1$-$C_4$-Halogenalkoxy, $C_1$-$C_4$-Alkylthio oder $C_1$-$C_4$-Halogenalkylthio;

Benzyloxy oder Benzylthio, wobei die aromatischen Reste ein bis fünf Halogenatome, einen Phenyl-, Phenoxy- oder Phenylthiorest und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

zur Bekämpfung von Schädlingen.

**2.** Verwendung von alpha-Arylacrylsäurederivaten der allgemeinen Formel I,

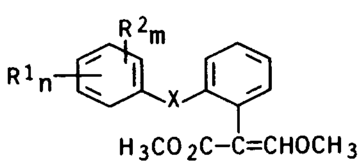

I

in der die Substituenten und Indizes folgende Bedeutung haben:

X
Ethenylen;

n
0 oder 1;

m

0, 1, 2 oder 3, wobei die Reste $R^2$ verschieden sein können, wenn m für 2 oder 3 steht;

$R^1$
Cyano;

$C_3$-$C_8$-Cycloalkyl, $C_5$-$C_8$-Cycloalkenyl oder $C_5$-$C_8$-Cycloalkdienyl, wobei diese Reste ein bis fünf Halogenatome, einen Phenyl-, Phenoxy- oder Phenylthiorest und/oder ein bis drei der folgenden Reste tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

$C_2$-$C_6$-Alkenyl, wobei dieser Rest ein bis fünf Halogenatome, einen Phenyl-, Phenoxy- oder Phenylthiorest und/oder ein bis drei der folgenden Reste tragen kann: $C_1$-$C_4$-Alkoxy, $C_1$-$C_4$-Halogenalkoxy, $C_1$-$C_4$-Alkylthio oder $C_1$-$C_4$-Halogenalkylthio;

$R^2$
Halogen;

$C_1$-$C_4$-Alkyl, $C_1$-$C_4$-Alkoxy, $C_2$-$C_6$-Alkenyloxy oder $C_5$-$C_{10}$-Alkdienyloxy, wobei diese Reste ein bis fünf Halogenatome, einen Phenyl-, Phenoxy-oder Phenylthiorest und/oder ein bis drei der folgenden Reste tragen können $C_1$-$C_4$-Alkoxy, $C_1$-$C_4$-Halogenalkoxy, $C_1$-$C_4$-Alkylthio oder $C_1$-$C_4$-Halogenalkylthio,

zur Bekämpfung von Schädlingen.

**3.** Verfahren zur Bekämpfung von Schädlingen, dadurch gekennzeichnet, daß man die Schädlinge und/oder ihren Lebensraum mit einer wirksamen Menge eines alpha-Arylacrylsäurederivats der allgemeinen Formel I gemäß Anspruch 1 behandelt.

**4.** Verfahren zur Bekämpfung von Schädlingen, dadurch gekennzeichnet, daß man die Schädlinge und/oder ihren Lebensraum mit einer wirksamen Menge eines alpha-Arylacrylsäurederivats der allgemeinen Formel I gemäß Anspruch 2 behandelt.